# EUROPEAN PATENT SPECIFICATION

(11) **EP 3 636 248 B1**
(45) Date of publication and mention of the grant of the patent: **11.12.2024**
(21) Application number: 19210949.4
(22) Date of filing: 07.04.2017
(51) Int. Cl.: A61K 8/73, A61Q 19/00, A61Q 19/08, A61K 8/04

(54) **TOPICAL COMPOSITIONS CONTAINING CROSS-LINKED GLYCOSAMINOGLYCANS**
TOPISCHE ZUSAMMENSETZUNGEN MIT VERNETZTEN GLYKOSAMINOGLYKANEN
COMPOSITIONS TOPIQUES CONTENANT DES GLYCOSAMINOGLYCANES RÉTICULÉS

(30) Priority: 08.04.2016 US 201662319847 P
(43) Date of publication of application: 15.04.2020
(62) Divisional of application: 17165566.5
(73) Proprietor: Johnson & Johnson Consumer Inc., Skillman, NJ 08558 (US)
(72) Inventor: FASSIH, Ali, Skillman, NJ New Jersey 08558 (US); PATURI, Jyotsna, Los Altos, CA California 94024 (US); WANGARI-TALBOT, Janet, Hillsborough, NJ New Jersey 08844 (US)
(74) Representative: Carpmaels & Ransford LLP

(56) References cited:
- EP-A1- 2 366 386
- WO-A1-2012/154505
- JP-A- 2007 252 212
- US-A1- 2010 098 764

## Description

### Field of the Invention

The present invention provides topical compositions comprising cross-linked sulfated glycosaminoglycans and methods of making and using the same for treating skin, for example for signs of skin aging.

### Background of the Invention

Glycosaminoglycans, or GAG's, are naturally occurring, high molecular weight polysaccharides. They are present in human cartilage, joint fluids, and skin tissue. They play roles in several biological processes in the body, such as moisturization and lubrication of tissues. Chondroitin sulfate, for example, is a sulfated glycosaminoglycan that is a copolymer of N-acetyl-D-galactosamine and D-glucuronic acid. It is commonly used to treat joint disorders as an oral supplement, injectable, or topical cream. Heparan sulfate, keratin sulfate and dermatan sulfate are other known sulfated glycosaminoglycans.

It would be desirable to deliver chondroitin sulfate and other sulfated glycosaminoglycans to treat cosmetic skin conditions, such as signs of aging or dry skin, by topical administration, which is convenient and painless. However, the stratum corneum of mammalian skin presents a formidable barrier to penetration. The ability of a substance to penetrate through the skin is inversely related to the thickness of the stratum corneum layer and the size of the substance. Large, polymeric molecules are difficult to administer topically. In addition, the negatively charged sulfate groups of sulfated glycosaminoglycans also reduce their skin penetration efficiencies.

Compositions and methods for the topical delivery of sulfated glycosaminoglycans in cross-linked, nanoparticle form have now been identified. Methods of treating skin using such compositions are also provided. Cross-linked sulfated glycosaminoglycan nanoparticles are capable of effective skin penetration alone or as a delivery system for other active agents.

EP 2366386 A1 describes a system for administering active ingredients comprising nanoparticles. JP 2007252212 describes a method for producing compositions comprising chondroitin sulfate, taurine or collagen. US 2010/0098764 A1 describes polysaccharide gel formulations including at least one inhibitor of polysaccharide degradation. WO 2012/154505 A1 describes stable aqueous compositions having dispersed therein hydrogel particles comprising polysaccharides.

### Summary of the Invention

The present invention provides a topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions, and wherein the composition further comprises a buffering agent selected from the group consisting of lactic acid, glycolic acid, citric acid, tartaric acid, gluconic acid, and gluconolactone.

The present invention also provides a topical composition comprising a cosmetically acceptable active ingredient carried in a matrix comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions, and wherein the composition further comprises a buffering agent selected from the group consisting of lactic acid, glycolic acid, citric acid, tartaric acid, gluconic acid, and gluconolactone.

The present invention further provides a method of treating signs of skin aging, comprising topically applying to skin in need of treatment for signs of skin aging a topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions. The method of treating signs of skin aging may be cosmetic.

The invention also provides a method of moisturizing skin, comprising topically applying to skin in need of moisturization treatment a topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions. The method of moisturizing skin may be cosmetic.

The invention also provides a method of making a topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions, wherein the method comprises dissolving the sulfated glycosaminoglycan in phosphate buffered saline to form a solution, and the drop-wise titration of a solution containing said ion into the solution of the sulfated glycosaminoglycan with continuous mixing.

### Detailed Description of the Invention

Unless defined otherwise, all technical and scientific terms used herein have the same meaning as commonly understood by one of ordinary skill in the art to which the invention belongs.

As used herein, "topically applying" means directly laying on or spreading on outer skin, the scalp, or hair, e.g., by use of the hands or an applicator such as a wipe, roller, or spray.

As used herein, "cosmetically acceptable" means that the ingredients the term describes are suitable for use in contact with tissues (e.g., the skin or hair) without undue toxicity, incompatibility, instability, irritation, allergic response, or the like.

As used herein, a "cosmetically acceptable active agent" is a compound (synthetic or natural) that has a cosmetic or therapeutic effect on the skin or hair.

Compositions of the present invention are suitable for treating signs of skin aging. As used herein, "signs of skin aging" includes the presence of lines and wrinkles, loss of elasticity, uneven skin, and blotchiness. In a particularly preferred embodiment, the sign of aging is the presence of lines and wrinkles and/or loss of elasticity.

As used herein, "treating signs of skin aging" refers to mitigating, reducing, preventing, improving, or eliminating the presence or signs of skin aging described above.

As used herein, "wrinkle" includes fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "loss of elasticity" includes loss of elasticity or structural integrity of the skin or tissue, including but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "uneven skin" means a condition of the skin associated with diffuse or mottled pigmentation, which may be classified as hyperpigmentation, such as post-inflammatory hyperpigmentation.

As used herein, "blotchiness" means a condition of the skin associated with redness or erythema.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically effective amount" means an amount of a physiologically active compound or composition sufficient for treating one or more signs of skin aging, but low enough to avoid serious side effects. The cosmetically effective amount of the compound or composition will vary with the particular condition being treated, the age and physical condition of the end user, the severity of the condition being treated/prevented, the duration of the treatment, the nature of other treatments, the specific compound or product/composition employed, the particular cosmetically-acceptable carrier utilized, and like factors.

Compositions of the invention are also useful for treating skin in need of moisturization. As used herein, "skin in need of moisturization" means a skin that is, but not limited to, lacking in moisture, lacking in sebum, cracked, dry, itchy, scaly, xerodermic, dehydrated, lacks suppleness, lacks radiance, dull, or lacks lipids.

Unless otherwise indicated, a percentage or concentration refers to a percentage or concentration by weight (i.e., % (W/W). Unless stated otherwise, all ranges are inclusive of the endpoints, e.g., "from 4 to 9" includes the endpoints 4 and 9.

Preferably, cross-linked sulfated glycosaminoglycan particle sizes are measured using SEM.

### Sulfated Glycosaminoglycans

The composition comprises one or more sulfated glycosaminoglycan which are cross-linked according to the invention. The glycosaminoglycan substrates used to create the cross-linked sulfated glycosaminoglycan may be selected from the group consisting of chondroitin sulfate, heparan sulfate, dermatan sulfate, and keratan sulfate. Mixtures of the foregoing may be used.

The cross-linked sulfated glycosaminoglycan may be fully or partially cross-linked. A mixture of linear and cross-linked sulfated glycosaminoglycans may also be used.

The sulfated glycosaminoglycan used to make the cross-linked sulfated glycosaminoglycan may be obtained from animal sources or bacterial sources.

In one embodiment, the cross-linked sulfated glycosaminoglycan is cross-linked chondroitin sulfate. The chondroitin sulfate may be animal-derived, such as from salmon, shark, bovine, or porcine origin. Animal-derived chondroitin sulfate is commercially available from Sigma Aldrich and other chemical suppliers.

In another embodiment, the chondroitin sulfate is bacterial-derived. Examples of bacterial-derived chondroitin sulfate include MYTHOCONDRO^{®}, commercially available from Gnosis S.p.A. Bacterial-derived chondroitin sulfate may be preferred as a cosmetically acceptable ingredient because its use avoids animal use and testing.

The cross-lined sulfated glycosaminoglycan is cross-linked with a monovalent, divalent, or trivalent cation, or any cationic species comprising monovalent or trivalent ions. The cross-linked sulfated glycosaminoglycan is cross-linked with an ion selected from the group consisting of calcium ions, strontium ions, and magnesium ions. In particular, the cross-linked sulfated glycosaminoglycan may comprise calcium ions.

The cross-linked sulfated glycosaminoglycan may be made by a variety of methods. For example it may be made by drop-wise titration of a solution containing the desired ion into a solution of the sulfated glycosaminoglycan with continuous mixing. The sulfated glycosaminoglycan may be dissolved in water or phosphate buffered saline. A salt containing the desired ion is added in slowly with constant stirring until a hazy suspension is achieved.

The degree of cross-linking and particle size may be adjusted by controlling the pH of the mixture and the speed of mixing. As such, the cross-linking may be altered by varying pH environment in use, such as with intradermal penetration. In one embodiment, the particles uncross-link when the pH environment of the skin is varied, releasing contents of the particle to the skin.

In one embodiment, the cross-linked sulfated glycosaminoglycan is in the form of nanoparticles. In one embodiment the nanoparticles have a particle size of about 100 nm to about 10 µm. In another embodiment, the nanoparticles have a particle size of about 500 nm to about 5 µm

In one embodiment, the topical composition comprises cross-linked sulfated glycosaminoglycan as an active ingredient itself.

### Cosmetically Acceptable Active Agents

Any cosmetically acceptable active agent may also be used in the composition. It may be loaded in the cross-linked sulfated glycosaminoglycan or present separately in the topical composition.

In a particular embodiment, the cosmetically acceptable active agent is loaded into cross-linked sulfated glycosaminoglycan, which forms a polymer matrix. This enables the cross-linked chondroitin sulfate to function as a delivery system for the active agent.

The cosmetically acceptable active agent may be loaded into the cross-linked sulfated glycosaminoglycan by entrapment.

The cosmetically acceptable active agent may be loaded into the cross-linked sulfated glycosaminoglycan by ionic binding.

In one embodiment, the cosmetically acceptable active agent may be a sulfate-containing agent which is ionically cross linked with the chondroitin sulfate polymer matrix.

Without wishing to be bound by theory, it is believed that cross-linked chondroitin sulfate may assist in the delivery of cosmetically acceptable active agents through the skin and preferably follicular structures within the skin given the size range of cross linked particles. This size range is ideal for permeation into follicular appendages and achieves sustained release benefits over time.

Cosmetically acceptable active agents include for example anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, agents for hair and/or skin conditioning, and other glycosaminoglycans such as hyaluronic acid.

The amount of cosmetically active agent in the composition may range from about 0.001% to about 20% by weight of the composition, e.g., about 0.005% to about 10% by weight of the composition, such as about 0.01% to about 5% by weight of the composition.

The cosmetically acceptable active agent may be selected for instance from hydroxy acids, benzoyl peroxide, D-panthenol carotenoids, retinoids such as retinol and retinyl palmitate, ceramides, polyunsaturated fatty acids, essential fatty acids, enzymes such as laccase, enzyme inhibitors, minerals, hormones such as estrogens, steroids such as hydrocortisone, 2-dimethylaminoethanol, copper salts such as copper chloride, peptides like argireline, syn-ake and those containing copper, coenzyme O10, amino acids such as proline, vitamins, lactobionic acid, acetyl-coenzyme A, niacin, riboflavin, thiamin, ribose, electron transporters such as NADH and FADH2, natural extracts such as from aloe vera, feverfew, oatmeal, dill, blackberry, princess tree, resorcinols such as 4-hexyl resorcinol, curcuminoids, and derivatives and mixtures thereof.

Examples of vitamins include, but are not limited to, vitamin A, vitamin B's such as vitamin B3, vitamin B5, and vitamin B12, vitamin C, vitamin K, and different forms of vitamin E like alpha, beta, gamma or delta tocopherols or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

### Topical Compositions

The compositions of the present invention are applied topically to human skin or hair. Accordingly, the composition further includes a cosmetically acceptable topical carrier. The carrier may be from about 50% to about 99.99%, by weight, of the composition (e.g., from about 80% to about 99%, by weight, of the composition). In a preferred embodiment of the invention, the cosmetically acceptable topical carrier includes water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain a variety of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

Compositions useful in the subject invention may be formulated as a solution comprising an emollient. Such compositions preferably contain from about 2% to about 50% of an emollient(s). As used herein, "emollients" refer to materials used for the prevention or relief of dryness, such as by preventing the transepidermal loss of water from the skin. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook"). Examples of particularly suitable emollients include vegetable oils, mineral oils, fatty esters, and the like.

A lotion can be made from such a solution. Lotions typically contain from about 1% to about 20% (e.g., from about 5% to about 10%) of an emollient(s) and from about 50% toabout 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

The composition of the present invention may include water or alternatively be anhydrous or be an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 2979-84.

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (e.g., a wax-based stick, soap bar composition, powder, or a wipe containing powder).

The compositions useful in the subject invention may contain, in addition to the aforementioned components, a wide variety of additional oil-soluble materials and/or water-soluble materials conventionally used in compositions for use on skin and hair, at their art-established levels.

Various other materials may also be present in the composition, as known in the art. These include humectants, pH adjusters, chelating agents (e.g., EDTA), fragrances, dyes, and preservatives (e.g., parabens).

The composition and formulations and products containing such compositions of the present invention may be prepared using methodology that is well known by an artisan of ordinary skill.

In one embodiment, the topical composition comprises a cross-linked sulfated glycosaminoglycan in an emulsion comprising an aqueous phase, oil phase, and non-ionic lipid phase.

The aqueous phase contains water.

The aqueous phase may also contain structuring agents such as carbomers or other thickeners, for example, xanthan gum, carageenan gum, polyacrylate-13; polyisobutene; polysorbate-20; polyacrylate-13/polyisobutylene/polysorbate-20 blends, and the like including mixtures thereof.

Preferably, the composition comprises a thickener and the thickener is hydroxyethyl acrylate/sodium acryloyldimethyl taurate copolymer.

The oil phase contains at least one cosmetically-acceptable oil.

As used herein, the term "oil" means a hydrophobic material that can aid in balancing the intermolecular forces to form micelle aggregates or to limit their sizes. Oils also serve as emollient ingredients to benefit product spreadibility, skin feel and delivery of hydrophobic active ingredients such as but not limited to, Vitamins D, E, K and A, and sunscreen filters.

Oils that are useful in the composition include a variety of hydrocarbon-based oils, silicones, fatty acid derivatives, glycerides, vegetable oils, vegetable oil derivatives, alkyl esters, wax esters, beeswax derivatives, sterols, and phospholipids and combinations thereof ranging from approximately 20% to 50%, based on the total weight of the composition.

Suitable hydrocarbon oils include petrolatum, mineral oil, micro-crystalline waxes, squalene and combinations thereof.

Silicone oils include dimethicone, dimethiconol, phenyl dimethicone and cyclic polysiloxanes and combinations thereof. Silicone oils having viscosities from about 0.5 to about 100,000 centistokes at 25° C. may also be useful in the composition.

Glycerides include castor oil, sunflower seed oil, coconut oil and derivatives, vegetable oils and derivatives, palm oil, jojoba oil, Shea butter, lanolin and combinations thereof.

Alkyl ester oils include, but are not limited to, isopropyl esters of fatty acids and esters of long chain fatty acids. More preferably, the following alkyl esters are useful: isopropyl palmitate, isopropyl myristate, myristyl myristate, isohexyl palmitate, decyl oleate, isononyl isononanoate and combinations thereof.

The non-ionic lipid phase comprises one or more non-ionic lipids, such as glyceryl monoesters having a fatty acid chain containing from about 3 to about 50 carbon atoms, and preferably from about 10 to about 18 carbon atoms; glyceryl diesters having a fatty acid chain containing from about 5 carbon atoms to about 25 carbon atoms, and preferably from about 10 carbon atoms to about 18 carbon atoms; alkoxylated alcohols; alkoxylated alkyl phenols; alkoxylated acids; alkoxylated amides; alkoxylated sugar derivatives; alkoxylated derivatives of natural oils or waxes; polyoxyethylene polyoxypropylene block copolymers; polyoxyethylene ether fatty acids having a fatty acid chain containing from about 10 carbon atoms to about 18 carbon atoms; steroids; fatty acid esters of alcohols where the fatty acid is straight or branched chain having from about 10 carbon atoms to about 20 carbon atoms and the alcohol is straight or branched chain having 1 to 10 carbon atoms; and mixtures thereof, wherein the alkoxylated lipids are alkoxylated with ethylene oxide or propylene oxide, with ethylene oxide being preferred.

Examples of suitable glyceryl monoesters include, but are not limited to, glyceryl caprate, glyceryl caprylate, glyceryl cocate, glyceryl erucate, glyceryl hydroxysterate, glyceryl isostearate, glyceryl lanolate, glyceryl laurate, glyceryl linolate, glyceryl myristate, glyceryl oleate, glyceryl PABA, glyceryl palmitate, glyceryl ricinoleate, glyceryl stearate, glyceryl thiglycolate, and mixtures thereof, with glyceryl laurate and glyceryl myristate being preferred.

Examples of suitable glyceryl diesters include, but are not limited to, glyceryl dilaurate, glyceryl dioleate, glyceryl dimyristate, glyceryl disterate, glyceryl sesuioleate, glyceryl stearate lactate, and mixtures thereof, with glyceryl dilaurate and glyceryl dimyristate being preferred.

Examples of suitable polyoxyethylene fatty ethers include, but are not limited to, polyoxyethylene cetyl/stearyl ether, polyoxyethylene cholesterol ether, polyoxyethylene laurate or dilaurate, polyoxyethylene stearate or distearate, polyoxyethylene lauryl or stearyl ether, and mixtures thereof, wherein the polyoxyethylene head group ranges from about 2 to about 100 groups. Preferred polyoxyethylene fatty ethers include polyoxyethylene stearyl ether, polyoxyethylene myristyl ether, and polyoxyethylene lauryl ether having from about 3 to about 10 oxyethylene units.

Examples of suitable steroids include, but are not limited to, cholesterol, betasitosterol, bisabolol, and mixtures thereof.

Examples of suitable fatty acid esters of alcohols include isopropyl myristate, aliphati-isopropyl n-butyrate, isopropyl n-hexanoate, isopropyl n-decanoate, isoproppyl palmitate, octyidodecyl myristate.

Exemplary alkoxylated alcohols useful as the nonionic lipid in the compositions of the invention have the structure shown in Formula I below:

R₅--(OCH₂CH₂)y--OH Formula I

wherein R₅ is a branched or unbranched alkyl group having from about 6 to about 22 carbon atoms and y is between about 4 and about 100, and preferably, between about 10 and about 100. A preferred alkoxylated alcohol is the species wherein Rs is a lauryl group and y has an average value of 23, which is known as laureth 23 and is available from ICI Americas, Inc. of Wilmington, Del. under the tradename "BRIJ 35."

Another exemplary alkoxylated alcohol is an ethoxylated derivative of lanolin alcohol. Lanolin alcohol is a mixture of organic alcohols obtained from the hydrolysis of lanolin. An example of an ethoxylated derivative of lanolin alcohol is laneth-10, which is the polyethylene glycol ether of lanolin alcohol with an average ethoxylation value of 10.

Another exemplary alkoxylated alcohol is polyoxypropylene polyoxyethylene alkyl ether, for example PPG-12-Buteth-16. This material is available from Amerchol Corp. of Edison, N.J. under the tradename, "UCON Fluid 50-HB-660."

Another type of non-ionic lipids includes alkoxylated alkyl phenols, for example nonoxynol-14"and is available under the tradename, "MAKON 14" from the Stepan Company of Northfield, III.

Another type of non-ionic lipids is alkoxylated acids, which are esters of an acid, most usually a fatty acid, with a polyalkylene glycol, for example PEG-8 laurate.

Another type of non-ionic lipids includes alkoxylated amides, for example PEG-6 cocoamide.

Another type of non-ionic lipids includes the alkoxylated sugar derivatives, for instance polysorbate 20, a mixture of laurate esters of sorbitol and sorbitol anhydrides, consisting predominately of the monoester, condensed with about 20 moles of ethylene oxide. This material is available under the tradename "TWEEN 20" from ICI Americas of Wilmington, Del.

Another example of an alkoxylated sugar derivative useful in the compositions of the invention is PEG-20 methylglucose sesquistearate, which is the polyethyleneglycol ether of the sesquiester of methyl glucose and stearic acid, contains an average of 20 moles of ethylene oxide, and is available under the tradename, "GLUCAMATE SSE-20" from the Amerchol Corp. of Edison, N.J.

Another type of non-ionic lipids includes the alkoxylated derivatives of natural oils and waxes. Examples of this class of material include PEG-40 lanolin, PEG-40 castor oil and PEG-40 hydrogenated castor oil.

Another type of non-ionic lipids includes polyoxyethylene polyoxypropylene block copolymers, for example Poloxamer 101 and Poloxamer 182.

Preferred nonionic lipids include polyoxyethylene fatty ethers, glyceryl diesters, and mixtures thereof. More preferred nonionic lipids include polyoxyethylene stearyl ether, polyoxyethylene myristyl ether, and polyoxyethylene lauryl ether, glyceryl dilaurate, glyceryl dimystate, glyceryl distearate, and mixtures thereof, whereby each ether has from about 5 to about 10 oxyethylene units.

In an embodiment wherein the reduction of skin irritation is a concern, it is preferable to use a nonionic lipid having a greater amount of carbon atoms on the hydrophilic head group moiety, or in the alternative, a nonionic lipid having a greater amount of carbon atoms on the hydrophobic fatty acid chain moiety. The former can be achieved by increasing the amount of carbon atoms on the head group of, for example, a polyoxyethylene-10-stearyl ether from about 10 carbon atoms to from about 15 to 20 carbon atoms. The latter can be achieved by increasing the amount of carbon atoms on the 12 carbon fatty acid tail of, for example, glyceryl diesters to from about 14 carbons to about 16 carbons.

The composition of the present invention includes, based upon the total weight of the composition, from about 1 percent to about 10 percent, and preferably from about 3 percent to about 7 percent of the nonionic lipid.

In a preferred embodiment, the non-ionic lipid phase comprises water, glyceride dilaurate, steareth-10, and glycerin.

In one embodiment the composition comprises chondroitin sulfate, lactic acid, steareth 10, glycerol dilaurate, and glycerin. The chondroitin sulfate may be linear chondroitin sulfate. The chondroitin sulfate may be cross-linked chondroitin sulfate. The chondroitin sulfate may be a mixture of linear and cross-linked chondroitin sulfate.

### pH

In one embodiment, the topical composition has a low pH. For example, the pH may be less than about 4 or less than about 3.3.

The topical composition comprises a buffering agent selected from the group consisting of lactic acid, glycolic acid, citric acid, malic acid, tartaric acid, gluconic acid, and gluconolactone. Preferably the buffering agent is lactic acid.

Typically, the composition contains about 3 to about 12, or about 4 to about 8, weight percent of buffering agent.

### Methods of Treating Skin

According to the invention signs of skin aging may be treated by topically applying to skin in need of treatment for signs of skin aging a topical composition comprising a cross-linked sulfated glycosaminoglycan and a cosmetically acceptable topical carrier.

In addition, skin may be moisturized by topically applying to skin in need of moisturization treatment a topical composition comprising a cross-linked sulfated glycosaminoglycan and a cosmetically acceptable topical carrier.

The following non-limiting examples further illustrate the invention.

### Example 1

Samples of cross-linked chondroitin sulfate were made according to the

Each of bovine trachea-derived and bacterial-derived chondroitin sulfate (CS) was used at the following amounts: 0.4%, 1.1%, 3.3% and 10% w/v. The bovine trachea-derived CS was obtained from Sigma-Aldrich. The bacteria-derived CS was obtained from Gnosis S.p.A.

The samples of chondroitin sulfate were dissolved in water or phosphate buffered saline (PBS) to produce 10 ml solutions. Then 10.1 M calcium chloride solution was added dropwise to each sample with stirring at 0.18 grams/gram of chondroitin sulfate. (For example for the 3.3% CS 10 ml solution, 40.8 ul of 10.1 M calcium chloride solution was added.) Samples made using CS in the PBS vehicle resulted in hazy suspensions (due to the ions on PBS), while the samples made using CS in water remained clear.

The resulting suspensions were analyzed by SEM for the presence of nanoparticles. Electron microscopy images confirmed the presence of particles in all samples. However, the samples made in PBS resulted in more particles than the samples made in water.

Solutions of linear chondroitin sulfate starting material in both the vehicles were also evaluated by SEM. In the absence of a positively charged ion, no nanoparticles formed and the solutions were clear compared to the hazy suspensions formed due to the presence of Ca²⁺.

Additional samples were made by adding all the CaCl₂ solution in one step. This resulted in larger, micron-sized aggregates of the particles, also confirmed by SEM.

### Example 2

*In vitro* skin penetration of linear and calcium cross-linked chondroitin sulfate using the samples listed in Table 1 below was measured as follows.

Chondroitin sulfate was obtained from Gnosis S.p.A.

Cross-linked chondroitin sulfate was made as described in Example 1 using PBS as the vehicle. PBS solution served as the control.

The penetration tests were performed using human epidermal equivalent tissues (MatTek Corporation, EpiDerm-Epi-200). The epidermal equivalent tissues were handled and cultured following the vendor's instructions. Prior to testing the well plates containing the EpiDerm tissue samples were equilibrated in a humidified 37°C, 5% CO2 incubator for 24 hours. Then for each sample, 50 ul of sample was topically applied on tissue, and then incubated in culture for 48 hours.

Culture media underneath the tissue samples were collected 48 hours post-treatment and measured for chondroitin sulfate volume using HPAEC-PAD assay after Trifluoroacetic Acid (TFA) hydrolysis.

The results are shown in Table 1.

**TABLE 1**

| SAMPLE | PENETRATION (Mg-L) |
|---|---|
| Untreated | 1.7 |
| PBS (Control) | 0 |
| 0.4% Gnosis CS in PBS (Comparative) | 5 |
| 1.1% Gnosis CS in PBS (Comparative) | 29.2 |
| 3.3% Gnosis CS in PBS (Comparative) | 334.5 |
| 10%Gnosis CS in PBS (Comparative) | 516 |
| 0.4% Gnosis CS in PBS+CaCl2 | 9.7 |
| 1.1% Gnosis CS in PBS+CaCl2 | 18.6 |
| 3.3% Gnosis CS in PBS+CaCl2 | 249.5 |
| 10% Gnosis CS in PBS+CaCl2 | 332.5 |

## Claims

1. A topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions, and wherein the composition further comprises a buffering agent selected from the group consisting of lactic acid, glycolic acid, citric acid, tartaric acid, gluconic acid, and gluconolactone.

2. The topical composition of claim 1, wherein the cross-linked sulfated glycosaminoglycan is selected from the group consisting of cross-linked chondroitin sulfate, cross-linked heparan sulfate, cross-linked keratin sulfate, and cross-linked dermatan sulfate.

3. The topical composition of claim 1 or claim 2, wherein the cross-linked sulfated glycosaminoglycan comprises cross-linked chondroitin sulfate.

4. The topical composition of any preceding claim, wherein the cross-linked sulfated glycosaminoglycan has a particle size of about 100 nm to about 10 µm.

5. The topical composition of any preceding claim further comprising a cosmetically acceptable active ingredient.

6. The topical composition of any preceding claim having a pH of less than about 4.

7. The topical composition of any preceding claim, wherein the sulfated glycosaminoglycan is bacteria-derived.

8. The topical composition of any preceding claim, wherein the sulfated glycosaminoglycan is bacteria-derived chondroitin sulfate.

9. A topical composition comprising a cosmetically acceptable active ingredient carried in a matrix comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions, and wherein the composition further comprises a buffering agent selected from the group consisting of lactic acid, glycolic acid, citric acid, tartaric acid, gluconic acid, and gluconolactone.

10. A method of making a topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions, wherein the method comprises dissolving the sulfated glycosaminoglycan in phosphate buffered saline to from a solution, and the drop-wise titration of a solution containing said ion into the solution of the sulfated glycosaminoglycan with continuous mixing.

11. A method of claim 10, wherein the topical composition is a topical composition of any one of claims 1 to 9.

12. A non-therapeutic method of treating signs of skin aging, comprising topically applying to skin in need of treatment for signs of skin aging a topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions.

13. A non-therapeutic method of moisturizing skin, comprising topically applying to skin in need of moisturization treatment a topical composition comprising a cross-linked sulfated glycosaminoglycan and a cosmetically acceptable topical carrier, wherein the sulfated glycosaminoglycan is cross-linked with ions selected from the group consisting of calcium ions, strontium ions, and magnesium ions.

## Patentansprüche

1. Topische Zusammensetzung, umfassend ein vernetztes sulfatiertes Glycosaminoglycan und einen kosmetisch annehmbaren topischen Träger, wobei das sulfatierte Glycosaminoglycan mit Ionen ausgewählt aus der Gruppe bestehend aus Calciumionen, Strontiumionen und Magnesiumionen vernetzt ist und wobei die Zusammensetzung ferner ein Puffermittel ausgewählt aus der Gruppe bestehend aus Milchsäure, Glycolsäure, Citronensäure, Weinsäure, Gluconsäure und Gluconolacton umfasst.

2. Topische Zusammensetzung nach Anspruch 1, wobei das vernetzte sulfatierte Glycosaminoglycan ausgewählt ist aus der Gruppe bestehend aus vernetztem Chondroitinsulfat, vernetztem Heparansulfat, vernetztem Keratinsulfat und vernetztem Dermatansulfat.

3. Topische Zusammensetzung nach Anspruch 1 oder Anspruch 2, wobei das vernetzte sulfatierte Glycosaminoglycan vernetztes Chondroitinsulfat umfasst.

4. Topische Zusammensetzung nach einem der vorstehenden Ansprüche, wobei das vernetzte sulfatierte Glycosaminoglycan eine Partikelgröße von etwa 100 nm bis etwa 10 µm aufweist.

5. Topische Zusammensetzung nach einem der vorstehenden Ansprüche, ferner umfassend einen kosmetisch annehmbaren Wirkstoff.

6. Topische Zusammensetzung nach einem der vorstehenden Ansprüche mit einem pH-Wert von weniger als etwa 4.

7. Topische Zusammensetzung nach einem der vorstehenden Ansprüche, wobei das sulfatierte Glycosaminoglycan aus Bakterien abgeleitet ist.

8. Topische Zusammensetzung nach einem der vorstehenden Ansprüche, wobei das sulfatierte Glycosaminoglykan aus Bakterien abgeleitetes Chondroitinsulfat ist.

9. Topische Zusammensetzung, umfassend einen kosmetisch annehmbaren Wirkstoff, der in einer Matrix getragen ist, die ein vernetztes sulfatiertes Glycosaminoglycan und einen kosmetisch annehmbaren topischen Träger umfasst, wobei das sulfatierte Glycosaminoglycan mit Ionen ausgewählt aus der Gruppe bestehend aus Calciumionen, Strontiumionen und Magnesiumionen vernetzt ist und wobei die Zusammensetzung ferner ein Puffermittel ausgewählt aus der Gruppe bestehend aus Milchsäure, Glycolsäure, Citronensäure, Weinsäure, Gluconsäure und Gluconolacton umfasst.

10. Verfahren zur Herstellung einer topischen Zusammensetzung, die ein vernetztes sulfatiertes Glycosaminoglycan und einen kosmetisch annehmbaren topischen Träger umfasst, wobei das sulfatierte Glycosaminoglycan mit Ionen ausgewählt aus der Gruppe bestehend aus Calciumionen, Strontiumionen und Magnesiumionen vernetzt ist, wobei das Verfahren Lösen des sulfatierten Glycosaminoglycans in phosphatgepufferter Kochsalzlösung, um eine Lösung zu bilden, und tropfenweises Titrieren einer Lösung, die das Ion enthält, in die Lösung des sulfatierten Glucosaminoglucans unter fortwährendem Rühren umfasst.

11. Verfahren nach Anspruch 10, wobei die topische Zusammensetzung eine topische Zusammensetzung nach einem der Ansprüche 1 bis 9 ist.

12. Nichttherapeutisches Verfahren zur Behandlung von Anzeichen von Hautalterung, umfassend topisches Aufbringen einer topischen Zusammensetzung, die ein vernetztes sulfatiertes Glycosaminoglycan und einen kosmetisch annehmbaren topischen Träger umfasst, wobei das sulfatierte Glycosaminoglycan mit Ionen ausgewählt aus der Gruppe bestehend aus Calciumionen, Strontiumionen und Magnesiumionen vernetzt ist, auf Haut mit Bedarf an Behandlung von Anzeichen von Hautalterung.

13. Nichttherapeutisches Verfahren zur Hautbefeuchtung, umfassend topisches Aufbringen einer topischen Zusammensetzung, die ein vernetztes sulfatiertes Glycosaminoglycan und einen kosmetisch annehmbaren topischen Träger umfasst, wobei das sulfatierte Glycosaminoglycan mit Ionen ausgewählt aus der Gruppe bestehend aus Calciumionen, Strontiumionen und Magnesiumionen vernetzt ist, auf Haut mit Bedarf an Hautbefeuchtungsbehandlung.

## Revendications

1. Composition topique comprenant un glycosaminoglycane sulfaté réticulé et un support topique cosmétiquement acceptable, dans laquelle le glycosaminoglycane sulfaté est réticulé avec des ions choisis dans le groupe constitué par les ions calcium, les ions strontium et les ions magnésium, et dans laquelle la composition comprend en outre un agent tampon choisi dans le groupe constitué par l'acide lactique, l'acide glycolique, l'acide citrique, l'acide tartrique, l'acide gluconique, et de la gluconolactone.

2. Composition topique selon la revendication 1, dans laquelle le glycosaminoglycane sulfaté réticulé est choisi dans le groupe constitué par le sulfate de chondroïtine réticulé, le sulfate d'héparane réticulé, le sulfate de kératine réticulé et le sulfate de dermatane réticulé.

3. Composition topique selon la revendication 1 ou la revendication 2, dans laquelle le glycosaminoglycane sulfaté réticulé comprend du sulfate de chondroïtine réticulé.

4. Composition topique selon l'une quelconque des revendications précédentes, dans laquelle le glycosaminoglycane sulfaté réticulé a une taille de particule d'environ 100 nm à environ 10 pm.

5. Composition topique selon l'une quelconque des revendications précédentes, comprenant en outre un ingrédient actif cosmétiquement acceptable.

6. Composition topique selon l'une quelconque des revendications précédentes ayant un pH inférieur à environ 4.

7. Composition topique selon l'une quelconque des revendications précédentes, dans laquelle le glycosaminoglycane sulfaté est dérivé de bactéries.

8. Composition topique selon l'une quelconque des revendications précédentes, dans laquelle le glycosaminoglycane sulfaté est le sulfate de chondroïtine dérivé de bactéries.

9. Composition topique comprenant un ingrédient actif cosmétiquement acceptable transporté dans une matrice comprenant un glycosaminoglycane sulfaté réticulé et un support topique cosmétiquement acceptable, dans laquelle le glycosaminoglycane sulfaté est réticulé avec des ions choisis dans le groupe constitué par les ions calcium, les ions strontium et les ions magnésium, et dans laquelle la composition comprend en outre un agent tampon choisi dans le groupe constitué par l'acide lactique, l'acide glycolique, l'acide citrique, l'acide tartrique, l'acide gluconique, et de la gluconolactone.

10. Procédé de préparation d'une composition topique comprenant un glycosaminoglycane sulfaté réticulé et un support topique cosmétiquement acceptable, dans lequel le glycosaminoglycane sulfaté est réticulé avec des ions choisis dans le groupe constitué par les ions calcium, les ions strontium et les ions magnésium, dans lequel le procédé comprend la dissolution du glycosaminoglycane sulfaté dans une solution saline tamponnée au phosphate pour former une solution, et le titrage goutte à goutte d'une solution contenant ledit ion dans la solution du glycosaminoglycane sulfaté avec mélange continu.

11. Procédé selon la revendication 10, dans lequel la composition topique est une composition topique selon l'une quelconque des revendications 1 à 9.

12. Procédé non thérapeutique de traitement de signes de vieillissement cutané, comprenant l'application topique sur la peau nécessitant un traitement pour des signes de vieillissement cutané d'une composition topique comprenant un glycosaminoglycane sulfaté réticulé et un support topique cosmétiquement acceptable, dans lequel le glycosaminoglycane sulfaté est réticulé avec des ions choisis dans le groupe constitué par les ions calcium, les ions strontium et les ions magnésium.

13. Procédé non thérapeutique d'hydratation de la peau, comprenant l'application topique sur la peau nécessitant un traitement d'hydratation d'une composition topique comprenant un glycosaminoglycane sulfaté réticulé et un support topique cosmétiquement acceptable, dans lequel le glycosaminoglycane sulfaté est réticulé avec des ions choisis dans le groupe constitué par les ions calcium, les ions strontium et les ions magnésium.
